(19) Europäisches Patentamt
European Patent Office
Office européen des brevets

(11) Publication number: **0 357 243 B1**

(12) **EUROPEAN PATENT SPECIFICATION**

(45) Date of publication of patent specification: **01.02.95**

(51) Int. Cl.6: **C07C 209/68**, C07C 211/05, C07C 51/02

(21) Application number: **89307927.7**

(22) Date of filing: **03.08.89**

(54) **The production of formate salts of nitrogenous bases.**

(30) Priority: **20.08.88 GB 8819835**
**05.07.89 GB 8915411**

(43) Date of publication of application:
**07.03.90 Bulletin 90/10**

(45) Publication of the grant of the patent:
**01.02.95 Bulletin 95/05**

(84) Designated Contracting States:
**BE DE FR GB IT NL**

(56) References cited:
**EP-A- 0 095 321**
**EP-A- 0 126 524**
**EP-A- 0 151 510**
**EP-A- 0 181 078**
**EP-A- 0 329 337**

(73) Proprietor: **BP Chemicals Limited**
**Britannic House**
**1 Finsbury Circus**
**London EC2M 7BA (GB)**

(72) Inventor: **Beevor, Robert George BP Chemicals Limited**
**Salt End**
**Hedon**
**Hull**
**HU12 8DS (GB)**
Inventor: **Gulliver, David Jeffrey BP Chemicals Limited**
**Salt End**
**Hedon**
**Hull**
**HU12 8DS (GB)**
Inventor: **Kitson, Melanie BP Chemicals Limited**
**Salt End**
**Hedon**
**Hull**
**HU12 8DS (GB)**
Inventor: **Sorrell, Robert Michael BP Chemicals Limited**
**Salt End**
**Hedon**
**Hull**
**HU12 8DS (GB)**

(74) Representative: **Barlow, Michael Thomas et al**
**BP INTERNATIONAL LIMITED**
**Patents & Agreements Division**
**Chertsey Road**
**Sunbury-on-Thames**
**Middlesex TW16 7LN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.09/3.3.3)

## Description

The present invention relates generally to the production of formate salts of nitrogenous bases and in particular to the production of formate salts of nitrogenous bases containing a tertiary nitrogen atom and to their conversion into formic acid.

The production of formate salts of nitrogenous bases containing a tertiary nitrogen atom is described, for example, in EP-A-0 095 321 and EP-A-0 151 510. EP-A-0 095 321 discloses a process for the production of a formate salt of a nitrogenous base in the presence of a solvent and as catalyst a soluble compound of a transition metal of Group VIII of the Periodic Table according to Mendeleef and separating the formate salt of the base from the reaction mixture. EP-A-0 151 510 discloses a process for the production of a formate salt of a nitrogenous base containing a tertiary nitrogen atom which process comprises reacting hydrogen and carbon dioxide with the nitrogenous base containing a tertiary nitrogen atom in the presence of a solvent and an effective amount of a catalyst characterised in that the catalyst comprises an inorganic or organometallic compound of rhodium and an organophosphorus compound. Both the aforesaid processes are operated in a single liquid phase, generally using a homogeneous catalyst, though heterogeneous catalysts may be employed.

The conversion of the formate salts of nitrogenous bases into formic acid is described in EP-A-0 126 524 and EP-A-0 181 078 for example. EP-A-0 126 524 discloses a process for the preparation of formic acid from a formate salt in which the formic acid is combined with a first base characterised in that (1) the formate salt is contacted with a second, weaker and less volatile base under conditions which cause the first base to be displaced by the second base, (2) the formate salt of the second base is subsequently separated from the reaction mixture and (3) the formate salt of the second base is finally decomposed to yield formic acid and the second base. EP-0 181 078 discloses an integrated process for the production of formic acid from carbon dioxide and hydrogen characterised in that

(a) in a first stage a nitrogenous base, carbon dioxide and hydrogen are reacted together in the presence of a catalyst to produce a formate salt of the nitrogenous base;

(b) in a second stage the catalyst is removed from the formate salt of the nitrogenous base and any low boilers and recycled to the first stage;

(c) in a third stage the formate salt of the nitrogenous base is recovered from the low boilers;

(d) in a fourth stage the formate salt of the nitrogenous base is reacted with a base having a high boiling point to produce the nitrogenous base and the formate salt of the base having a high boiling point;

(e) in a fifth stage the formate salt of the base having a high boiling point is decomposed to the higher boiling base and formic acid.

In the first stage (a) of the process of EP-A-0181078 a high-boiling solvent is generally employed. The catalyst is generally a soluble Group VIII transition metal compound. In the second stage (b) of the process the catalyst and the high-boiling solvent is removed from the product of the first stage comprising unreacted materials, the formate salt of the nitrogenous base and catalyst in the high-boiling solvent. In a preferred arrangement the second stage comprises an evaporator wherein (i) the catalyst and high-boiling solvent are separated and recycled to the first stage reactor, and (ii) gaseous components are separated and recycled. A problem can occur in the operation of the evaporator in that under the conditions of elevated temperature and low pressure prevailing therein the presence of catalyst together with the formate salt of the nitrogenous base can cause the reverse reaction to occur, i.e. the decomposition of the formate salt of the nitrogenous base to the nitrogenous base, carbon dioxide and hydrogen, thereby decreasing the yield of the desirable formate salt. This problem is not restricted to operation using an evaporator for separation of the catalyst and high-boiling solvent, but may be encountered in any separation in which the catalyst remains in contact with the formate salt under conditions facilitating formate salt decomposition.

Solutions to the problem of formate salt decomposition in the presence of the catalyst used in its preparation have involved the addition of a compound which inactivates the catalyst either temporarily or permanently. Temporary inactivation during catalyst removal is preferred because the catalyst is re-usable on reactivation. Thus, our copending European application No. 89301270.8 describes the addition of a carboxylic acid or a salt thereof, an oxidant and carbon monoxide respectively as the inhibitor.

We have now found an alternative process for the production of formate salts of nitrogenous bases which also offers a different solution to the problem of formate salt decomposition during the catalyst separation step of the aforesaid integrated process for the production of formic acid.

Accordingly, the present invention provides a process for the production of a formate salt of a nitrogenous base which process comprises reacting hydrogen and carbon dioxide with a nitrogenous base in the presence of a catalyst comprising an inorganic or organometallic compound of a metal of Group VIII of the Periodic Table and a compound of an element of Group VA of the Periodic Table of the Elements -

wherein the process is operated in a two phase system comprising two substantially immiscible solvents, a first solvent which preferentially dissolves the catalyst and a second solvent which preferentially dissolves the formate salt of the nitrogenous base, and separating the first solvent phase containing dissolved catalyst from the second solvent phase containing dissolved formate salt of the nitrogenous base.

A prime requirement of any solvent for use in the process of the invention is that it must be inert under the reaction conditions. For this reason it is preferred to avoid the use of chlorinated solvents, ketones and aldehydes.

As the first solvent which preferentially dissolves the catalyst there may suitably be used an inert organic solvent. Suitable inert organic solvents include both aliphatic, for example paraffinic, and aromatic hydrocarbon solvents. Examples of suitable organic solvents include heptane and toluene. As the first solvent there is preferably used a hydrocarbyl phosphine, suitably of the formula:-

either

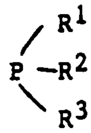

(I)

or

$R^1R^2P(CH_2)_nPR^3R^4$ (II)

or

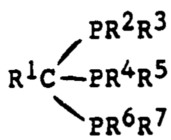

(II)

wherein $R^1$ to $R^7$ are independently either hydrogen or hydrocarbyl groups containing from 1 to 20 carbon atoms and n is an integer from 1 to 10. In addition, any two of $R^1$ to $R^7$ may together form an organic cyclic ring system bound to phosphorus. Suitably the hydrocarbyl group may be an alkyl, cycloalkyl, aryl or alkaryl group. Alternatively, organophosphorus compounds of the type $C_6H_5P[CH_2CH_2P(C_6H_5)_2]_2$ (triphos) may be employed. An example of a compound having the formula (I) is tri-n-butyl phosphine. An example of a compound having the formula (II) is 1,2-diphenylphosphinoethane $(C_6H_5)_2P(CH_2)_2P(C_6H_5)_2$ and an example of a compound having the formula (III) is $CH_3C[P(C_6H_5)_2]_3$.

Preferred hydrocarbyl phosphine solvents useful in the performance of the present invention include tri-n-butylphosphine.

As the second solvent which preferentially dissolves the formate salt of the nitrogenous base there may suitably be used either water, glycerol or water in admixture with a solvent comprising an alcohol, a glycol, a polyol, a sulpholane or a mixture of at least two thereof. A preferred second solvent is water.

Commercially available hydrogen may be used, with or without further purification.

The carbon dioxide may either be carbon dioxide itself, which is widely available on an industrial scale, or a carbonate or a bicarbonate or a mixture thereof. Carbon dioxide may be used as a gas or as a liquid or as a solid, preferably as a gas. Using carbon dioxide gas as the source of carbon dioxide it is preferred to use partial pressures of carbon dioxide and hydrogen which are as high as is practicable and economic. The use of high partial pressures of hydrogen is desirable because the reaction rate and yield of the formate salt increase as the partial pressure increases. The partial pressure of carbon dioxide is less critical but suitably the carbon dioxide partial pressure may be up to 60 bar and the hydrogen partial pressure up to 250 bar. Small amounts of impurities in the carbon dioxide and hydrogen can be tolerated.

Suitably the partial pressure of carbon dioxide is from 10 to 50 bar and that of hydrogen from 10 to 150 bar. The ratio of the partial pressure of hydrogen to that of carbon dioxide is preferably at least 1:1 more preferably at least 1.5:1.

The nitrogenous base may be a primary, secondary or tertiary amine, which may be substituted by, for example hydroxyl groups, for example alkanolamines. Preferred are nitrogenous bases containing a tertiary nitrogen atom, which may suitably be of the formula:-

either

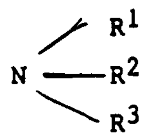

(IV)

or

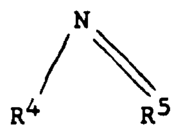

(V)

wherein in the formulae, $R^1$, $R^2$ and $R^3$, which may be the same or different, are hydrocarbyl groups or substituted hydrocarbyl groups or any two or all of $R^1$, $R^2$ and $R^3$ may form part of a ring, $R^4$ is a hydrocarbyl group or substituted hydrocarbyl group and $R^5$ is a divalent organic group or $R^4$ and $R^5$ may form part of a ring. Suitably the hydrocarbyl group is an aliphatic, cycloaliphatic, aryl or alkaryl group. Substituted hydrocarbyl groups may contain for example nitrogen or oxygen. Preferably the nitrogenous base containing a tertiary nitrogen atom is a trialkylamine, even more preferably a lower trialkylamine, for example a $C_1$ to $C_{10}$ trialkylamine. Examples of suitable trialkylamines are trimethylamine, triethylamine, tripropylamine and tributylamine. Examples of other nitrogenous bases which may be employed are amidines, e.g. 1,8-diazabicyclo[5.4.0]undec-7-ene (DBU) and 1,4-diazabicyclo[2.2.2]octane (DABCO). Mixtures of nitrogenous bases may be used if so desired. The nitrogenous base is suitably added in amounts which produce a concentration in the range 1 to 50 mole % based on the total reaction mixture.

The formate salt produced by the present process comprises a formate anion and a cation derived from the nitrogenous base by protonation. Thus, for example when the nitrogenous base used is triethylamine, the formate salt produced is triethylammonium formate.

As catalyst there is used an inorganic or organometallic compound of a metal of Group VIII of the Periodic Table and a compound of an element of Group VA of the Periodic Table. For the avoidance of doubt the Periodic Table used throughout this specification is that of Cotton and Wilkinson. Suitable Group VIII metals include iron, cobalt, nickel, ruthenium, rhodium, palladium, iridium or platinum. Preferred metals of Group VIII are rhodium and ruthenium. Mixtures of compounds of different Group VIII metals may also be used if desired. The metal may be added in any convenient form whether soluble or insoluble in the initial reaction medium, but preferably in a soluble form. Thus, the metal may be added in the form of a simple salt, for example the halide, nitrate, sulphate or acetylacetonate or in the form of an organometallic complex of the metal or as the elemental metal.

Suitably there is used a compound of an element of Group VA of the Periodic Table, for example a compound of nitrogen, phosphorus, arsenic, antimony or bismuth. Preferably there is used an organophosphorus compound. It will be appreciated that the hydrocarbyl phosphine employed as the first solvent as hereinbefore described may serve also as a component of the catalyst. Alternatively, the catalyst may utilise a different organophosphorus compound. Any organophosphorus compound of the formulae (I) to (III) may be employed. A preferred organophosphorus compound is tri-n-butylphosphine.

The catalyst may be formed 'in situ' by reacting a metal compound with the organophosphorus compound or the catalyst may be pre-synthesised and isolated before use in the process.

The ratio of the organophosphorus compound to the Group VIII metal may vary over a wide range.

The process may be operated at a temperature in the range from 15 to 200 °C, preferably from 30 to 120 °C.

The process may be operated batchwise or continuously.

At the completion of the reaction using a two-phase system the catalyst is mainly in one phase and the formate salt of the nitrogenous base is mainly in another phase. The catalyst is recovered from the formate salt by separating the phases, which can be accomplished in known manner, for example by decantation, to provide a catalyst-rich solution which may be recycled to the reaction and a formate salt-rich solution, from which the formate salt may be recovered. An advantage of the process of the invention is that the catalyst is not in contact with the formate salt during its separation therefrom and thereby the problem of formate salt decomposition in the presence of the catalyst can be avoided. The process can therefore be utilised to

advantage in the integrated process for the production of formic acid.

In another aspect therefore the present invention provides a process for the production of formic acid which comprises the steps of:-

(1) reacting hydrogen, carbon dioxide and a first nitrogenous base in the presence as catalyst of an inorganic or organometallic compound of a metal of Group VIII of the Periodic Table to produce a formate salt of the first nitrogenous base in a two-phase liquid system comprising two substantially immiscible solvents, the first solvent being one which preferentially dissolves the catalyst and the second solvent being one which preferentially dissolves the formate salt of the first nitrogenous base,

(2) separating the first solvent phase containing dissolved catalyst from the second solvent phase containing dissolved formate salt of the first nitrogenous base,

(3) recycling the first solvent containing dissolved catalyst in whole or in part to step (1),

(4) reacting the formate salt of the first nitrogenous base, either dissolved in the second solvent or separated therefrom, with a second nitrogenous base which is (i) weaker than the first base, and (ii) is less volatile than the first base under conditions to cause the second base to displace the first base and thereby form a formate salt of the second nitrogenous base which is thermally decomposable at a temperature higher than the boiling point of the first base,

(5) separating the formate salt of the second nitrogenous base from the first nitrogenous base, and

(6) thermally decomposing the formate salt of the second nitrogenous base separated in step (5) to yield formic acid and the second nitrogenous base.

In step (1) of the aforesaid process the first nitrogenous base is preferably a nitrogenous base containing a tertiary nitrogen atom as hereinbefore described. The hydrogen, carbon dioxide and catalyst are as hereinbefore described. The first and second solvents are as hereinbefore described, preferred being toluene/water.

In step (2) the first solvent phase may be separated from the second solvent phase by means known in the art, for example by decantation. It is an advantage of using a mixture of an organic solvent, for example toluene, and water that the catalyst partitions into the uppermost organic layer and the formate salt into the lowermost water layer, thereby facilitating separation by, for example, a weir arrangement.

In step (4), the formate salt of the first nitrogenous base either dissolved in the second solvent or separated therefrom is reacted with a second nitrogenous base. The second solvent containing dissolved formate salt of the first nitrogenous base may be treated with the first solvent before separation or reaction to extract into the first solvent any residual catalyst present therein. It is preferred to separate the formate salt of the first nitrogenous base from the second solvent before reaction with the second nitrogenous base. This may suitably be accomplished by distillation, there being little risk of decomposition in the absence of catalyst provided that the boiling point of the second solvent is below the decomposition temperature of the formate salt under the pressure employed.

The second nitrogenous base is selected so that

(1) it is weaker than the nitrogenous base used in the first stage of the process

(2) its formate salt is thermally decomposable at a temperature higher than the boiling point of the nitrogenous base used in the first stage of the process, and

(3) it is less volatile than the nitrogenous base used in the first stage of the process.

It will be seen from these criteria that the exact choice of such a base will depend upon which nitrogenous base is used in the first stage of the process.

Preferably the second base has a $pK_a$ in the range 4.0 to 9.0 and is an imidazole of the general formula:

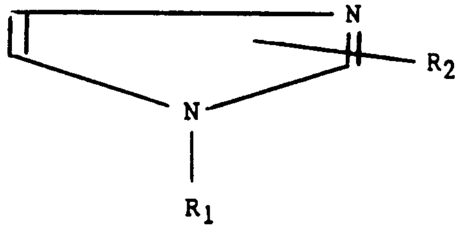

(VI)

where $R_1$ is a monovalent hydrocarbon group containing 1 to 12 carbon atoms and $R_2$ is a hydrogen atom or an $R_1$ group, the total number of carbon atoms in $R_1$ and $R_2$ conveniently being not more than 20 and preferably from 4 to 12.

Suitable hydrocarbon radicals on the imidazole derivatives (I) are, in general, alkyl groups of 1 to 8 carbon atoms, cyclopentyl, cyclohexyl, phenyl and methylphenyl groups. Amongst the above, imidazole derivatives where $R^1$ is n-1-alkyl of 4 to 10 carbon atoms and $R^2$ is hydrogen or methyl are particularly suitable. Examples of such compounds are 1-(n-1-butyl)-imidazole (pKa 5.9), 1-(n-1-pentyl)-imidazole (pKa 5.9), 1-(n-1-decyl)-imidazole (pKa 5.75), 1-(n-1-butyl)-2-methylimidazole (pKa 7.0) and 1-(n-1-pentyl)-2-methylimidazole (pKa 6.85).

In addition to imidazoles, quinoline and other heterocyclic nitrogenous bases can be used.

For a definition of the pKa values, which are a measure of the base strength, reference may be made, for example to Landoldt-Bornstein, 6th edition, 7th part volume II, page 900 et seq.

Thereafter, steps (5) and (6) may suitably be carried out in the manner described in the aforesaid EP-A-0 126 524, the content of which is incorporated herein by reference.

The invention will now be described by reference to the following Examples. In these Examples, the rate of reaction refers to the rate of production of the formate salt (moles/hr), averaged over the initial 75% of the reaction, divided by the weight of reaction solutions (Kgs) charged. The conversion to formate salt was calculated according to the following equation:-

$$\frac{\text{moles of formate produced}}{\text{moles of nitrogenous base added}} \times 100 = \text{conversion}$$

## Example 1

Into an autoclave of 300 ml capacity made of stainless steel and fitted with a rotary stirrer were charged 36.3g triethylamine, 27.15 g toluene, 20.1 g water, 2.33 g tri-n-butyl phosphine and 0.2047 g ruthenium trichloride. The toluene and water formed a two-phase system containing the catalyst, the phosphine ligand and the triethylamine. The autoclave was closed and carbon dioxide introduced until a steady pressure of 28 bar was obtained. The system was then heated until a steady temperature of 80 °C was achieved and at this point hydrogen was added to give a total pressure of 96 bar. At the end of the reaction period the autoclave was cooled to room temperature and the excess pressure discharged. The product solution formed two phases which were separated using a separating funnel. Formate analysis was carried out on each phase by hydrolysing with Amberlyst ion exchange resin followed by base titration. The data is summarised in Table 1. The rate of reaction was 0.22 mol $kg^{-1}h^{-1}$ with an overall conversion to triethylammonium formate of 41.5%.

## Example 2

Into the autoclave used for Example 1, following the same method, were charged 36.1 g triethylamine, 26.3 g toluene, 20.0 g water, 2.3 g tri-n-butyl phosphine and 0.2447 g of rhodium trichloride. The autoclave was closed and carbon dioxide introduced until a steady pressure of 28 bar was obtained. The system was then heated to 40 °C and hydrogen was added to give a total pressure of 96 bar. The rate of reaction was 0.07 mol $kg^{-1}h^{-1}$ with an overall conversion to triethylammonium formate of 18.3%.

## Example 3

Into the autoclave used for Example 1, following the same method, were charged 36 g triethylamine, 25.5 g toluene, 19.5 g water, 0.2628 g triphenylphosphine and 0.2302 g $[Rh(PPh_3)_3Cl]$. The autoclave was closed and carbon dioxide introduced until a steady pressure of 28 bar was obtained. The system was then heated to 40 °C and hydrogen was added to give a total pressure of 96 bar. The rate of reaction was 0.06 mol $kg^{-1}h^{-1}$ with an overall conversion to triethylammonium formate of 11.8%.

## Example 4

Into the autoclave used for Example 1, following the same method, were charged 36.5 g triethylamine, 26.8 g toluene, 20.2 g water and a 1:1 mixture of $[RuCl_3(PBu_3{}^n)_2]_2/[Ru_2Cl_5(PBu_3{}^n)_4]$. The autoclave was closed and carbon dioxide introduced until a steady pressure of 28 bar was obtained. The system was then heated to 80 °C and hydrogen was added to give a total pressure of 96 bar. The rate of reaction was 0.34 mol $kg^{-1}h^{-1}$ with an overall conversion to triethylammonium formate of 42.7%.

Example 5

Into the autoclave used for Example 1, following the same method, were charged 36.4 g triethylamine, 25.5 g toluene, 19.8 g water, 0.2627 g triphenylphosphine and 0.4805 g of [$RuCl_2(PPh_3)_3$]. The autoclave was closed and carbon dioxide introduced until a steady pressure of 28 bar was obtained. The system was then heated to 80 °C and hydrogen was added to give a total pressure of 96 bar. The rate of reaction was 1.72 mol $kg^{-1}h^{-1}$ with an overall conversion to triethylammonium formate of 56.9%.

Example 6

Into the autoclave similar to that described in Example 1, except of 100 mml capacity and following the method of Example 1, were charged 18.0 g triethylamine, 13.6 g toluene, 10.1 g water and 0.3154 g [$RuCl_3$-($PMe_2Ph)_3$]. The autoclave was closed and carbon dioxide introduced until a steady pressure of 28 bar was obtained. The system was then heated to 80 °C and hydrogen was added to give a total pressure of 96 bar. The rate of reaction was 2.45 mol $kg^{-1}h^{-1}$ with an overall conversion to triethylammonium formate of 70.8%.

Example 7

Into the autoclave used for Example 1, following the same method, were charged 36.7 g triethylamine, 25:3 g toluene, 20.1 g water and 1.1084 g [$RuCl_2$ (p-tolyl)$_3$P $_3$]. The autoclave was closed and carbon dioxide introduced until a steady pressure of 28 bar was obtained. The system was then heated to 80 °C and hydrogen was added to give a total pressure of 96 bar. The rate of reaction was 3.61 mol $kg^{-1}h^{-1}$ with an overall conversion to triethylammonium formate of 64.5%.

Example 8

Into the autoclave used for Example 6, following the method of Example 1, were charged 18.9 g triethylamine, 13.6 g toluene, 10.1 g water and 0.6146 g [$RuCl_3(PMe_2Ph)_3$]. The autoclave was closed and carbon dioxide introduced until a steady pressure of 28 bar was obtained. The system was then heated to 80 °C and hydrogen was added to give a total pressure of 96 bar. The rate of reaction was 7.4 mol $kg^{-1}h^{-1}$ with an overall conversion to triethylammonium formate of 68.5%.

Example 9

Into the autoclave used for Example 1 following the same method, were charged 36.9 g triethylamine, 20.2 g heptane, 20.2 g water and 0.6327 g [$RuCl_3(PMe_2Ph)_3$]. The autoclave was closed and carbon dioxide introduced until a steady pressure of 28 bar was obtained. The system was then heated to 80 °C and hydrogen was added to give a total pressure of 96 bar. The rate of reaction was 2.31 mol $kg^{-1}h^{-1}$ with an overall conversion to triethylammonium formate of 65.9%.

Example 10

Into the autoclave used for Example 6, following the method of Example 1, were charged 18.5 g triethylamine, 14.5 g toluene, 10.0 g water and 0.3130 g [$RuCl_3(PMe_2Ph)_3$]. The autoclave was closed and carbon dioxide introduced until a steady pressure of 28 bar was obtained. The system was then heated to 100 °C and hydrogen was added to give a total pressure of 96 bar. The rate of reaction was 6.59 mol $kg^{-1}h^{-1}$ with an overall conversion to triethylammonium formate of 52.1%.

Example 11

Into the autoclave used for Example 6 using the method of Example 1 was charged 13.7 g of the toluene phase from Example 10. This contained the catalyst and 4.9 g of triethylamine ascertained by acid titration. A further 14.8 g triethylamine and 10.0 g water were added to make up a standard charge. The autoclave was closed and carbon dioxide introduced until a steady pressure of 28 bar was obtained. The system was then heated to 100 °C and hydrogen was added to give a total pressure of 96 bar. The rate of reaction was 10.99 mol $kg^{-1}h^{-1}$ with an overal conversion to triethylammonium formate of 48.2%.

Example 12

Into the autoclave used for Example 6 using the method of Example 1 was charged 8.8 g of the toluene phase from Example 11. This contained the catalyst and 2.8 g of triethylamine ascertained by acid titration. A further 15.9 g of triethylamine and 10.0 g of water were added to make up a standard charge. The autoclave was closed and carbon dioxide introduced until a steady pressure of 28 bar was obtained. The system was then heated to 100 °C and hydrogen was added to give a total pressure of 96 bar. The rate of reaction was 11.80 mol $kg^{-1}h^{-1}$ with an overall conversion to triethylammonium formate of 50.0%.

Example 13

Into the autoclave used for Example 6, following the method of Example 1, were charged 17.83 g triethylamine, 13.82 g toluene, 16.93 g glycerol and 0.4627 g $[RuCl_2(PPh_3)_3]$. The autoclave was closed and carbon dioxide introduced until a steady pressure of 28 bar was obtained. The system was then heated to 80 °C and hydrogen was added to give a total pressure of 96 bar. The rate of reaction was 2.16 mol $kg^{-1}h^{-1}$ with an overall conversion to triethylammonium formate of 46.7%.

TABLE 1

| Example No. | Catalyst | mmoles Catalyst | Rate (mol $kg^{-1}h^{-1}$) | Triethylamine Conversion (%) | Triethylamine Formate Concentration (mmol $g^{-1}$) | | Metal Partitioning Ratio |
|---|---|---|---|---|---|---|---|
| | | | | | Aqueous Phase | Organic Phase | Organic:Aqueous |
| 1 | $RuCl_3/PBu^n_3$ | 0.99 | 0.22 | 41.5 | 2.622 | 0.018 | |
| 2 | $RhCl_3/PBu^n_3$ | 1.00 | 0.07 | 18.30 | 1.124 | 0.019 | 29:1 |
| 3 | $[Rh(PPh_3)_3Cl]$ | 0.25 | 0.06 | 11.8 | 0.871 | 0.006 | |
| 4 | $[RuCl_3(PBu^n_3)_2]_2$/ $[Ru_2Cl_5(PBu^n_3)_4]$ | 1.00 | 0.34 | 42.7 | 2.721 | 0.025 | 77:1 |
| 5 | $[RuCl_2(PPh_3)_3]$ | 0.50 | 1.72 | 56.9 | 3.275 | 0.023 | 73:1 |
| 6 | $[RuCl_3(PMe_2Ph)_3]$ | 0.51 | 2.45 | 70.8 | 3.843 | 0.027 | |
| 7 | $[RuCl_2$ (p-tolyl)$_3$P 3] | 1.02 | 3.61 | 64.5 | 3.818 | | |
| 8 | $[RuCl_3(PMe_2Ph_3]$ | 0.99 | 7.40 | 68.5 | 4.04 | 0.047 | |
| 9 | $[RuCl_3(PMe_2Ph)_3]$ | 1.02 | 2.31 | 65.9 | 3.707 | 0.047 | |
| 10 | $[RuCl_3(PMe_2Ph)_3]$ | 0.50 | 6.59 | 52.1 | 3.588 | 0.041 | 45:1 |
| 11 | $[RuCl_3(PMe_2Ph)_3]$ | 0.44 | 10.99 | 48.2 | 3.414 | 0.029 | |
| 12 | $[RuCl_3(PMe_2Ph)_3]$ | 0.34 | 11.80 | 50.0 | 3.148 | 0.044 | |
| 13 | $[RuCl_2(PPh_3)_3]$ | 0.50 | 2.16 | 46.7 | 2.829 | 0.036 | |

Example 14

Into an autoclave of 300ml capacity made of stainless steel and fitted with a rotary stirrer were charged under strictly anaerobic conditions the following degassed materials:- 28.72g triethylamine, 49.21g tri-n-butyl phosphine, 31.24g water and 0.3011g rhodium trichloride trihydrate. The autoclave was closed and carbon dioxide introduced until a steady pressure of 28 bar was obtained. The system was then heated until a steady temperature of 100 °C was achieved and at this point hydrogen was added to give a total pressure of 96 bar. At the end of the reaction period the autoclave was cooled to room temperature, the excess pressure discharged and the products collected again under strictly anaerobic conditions. The product solution formed two phases which were separated under anaerobic conditions via cannula transfer to a separate vessel. Formate analysis was carried out on each phase by hydrolysing with Amberlyst ion exchange resin followed by base titration. The data is summarised in Table 2. The rate of the reaction was 5.32 mol $kg^{-1}$ $h^{-1}$ with an overall conversion to triethylammonium formate of 68.4%.

Example 15

Into the autoclave used for Example 14 using the method of Example 14 was charged 49.84g of the tri-n-butyl phosphine (TNBP) phase from Example 14. This contained the catalyst and 0.90g of triethylamine ascertained by acid titration. A further 25.27g of degassed triethylamine and 31.56g of degassed water were then added under strictly anaerobic conditions to make up a standard charge. The autoclave was closed and carbon dioxide introduced until a steady pressure of 28 bar was obtained. The system was then heated to 100 °C and hydrogen added to give a total pressure of 96 bar. The rate of reaction was 6.83 mol $kg^{-1}$ $h^{-1}$ with an overall conversion to triethylammonium formate of 61.9%.

Example 16

Into the autoclave used for Example 14 using the method of Example 14 was charged 45.34g of the TNBP phase from Example 15. This contained the catalyst and 1.56g of triethylamine ascertained by acid titration. A further 24.58g of degassed triethylamine and 30.55g of degassed water were then added to make up a standard charge. The autoclave was closed and carbon dioxide introduced until a steady pressure of 28 bar was obtained. The system was then heated to 100 °C and hydrogen added to give a total pressure of 96 bar. The rate of reaction was 7.70 mol $kg^{-1}$ $h^{-1}$ with an overall conversion to triethylammonium formate of 73.2%.

Example 17

Into the autoclave used for Example 14 using the method of Example 14 was charged 42.62g of the TNBP phase from Example 16. This contained the catalyst and 1.18g of triethylamine ascertained by acid titration. A further 24.42g of degassed triethylamine and 30.58g of degassed water were then added to make up a standard charge. The autoclave was closed and carbon dioxide introduced until a steady pressure of 28 bar was obtained. The system was then heated to 100 °C and hydrogen added to give a total pressure of 96 bar. The rate of reaction was 8.00 mol $kg^{-1}$ $h^{-1}$ with an overall conversion to triethylammonium formate of 75.5%.

Example 18

Into the autoclave used for Example 14 using the method of Example 14, were charged 29.35g triethylamine, 48.13g tri-n-butyl phospine, 30.23g water and 0.295g ruthenium trichloride trihydrate. The autoclave was closed and carbon dioxide introduced until a steady pressure of 28 bar was obtained. The system was then heated to 100 °C and hydrogen was added to give total pressure of 96 bar. The rate of reaction was 3.71 mol $kg^{-1}$ with an overall conversion to triethylammonium formate of 53.8%.

Example 19

Into the autoclave used for Example 14 using the method of Example 14 was charged 46.74g of the TNBP phase from Example 18. This contained the catalyst and 1.28g of triethylamine ascertained by acid titration. A further 25.57g of degassed triethylamine and 31.21g of degassed water were then added to make up a standard charge. The autoclave was closed and carbon dioxide introduced until a steady pressure of 28 bar was obtained. The system was then heated to 100 °C and hydrogen added to give a total pressure of 96 bar. The rate of reaction was 2.31 mol $kg^{-1}$ $h^{-1}$ with an overall conversion to triethylammonium formate of 60.3%.

Example 20

Into the autoclave used for Example 14 using the method of Example 14 was charged 44.64g of the TNBP phase from Example 19. This contained the catalyst and 1.17g of triethylamine ascertained by acid titration. A further 25.23g of degassed triethylamine and 30.88g of degassed water were then added to make up a standard charge. The autoclave was closed and carbon dioxide introduced until a steady pressure of 28 bar was obtained. The system was then heated to 100°C and hydrogen added to give a total pressure of 96 bar. The rate of reaction was 0.69 mol $kg^{-1}$ $h^{-1}$ with an overall conversion to triethylammonium formate of 64.8%.

TABLE 2

| Example No. | Catalyst | mmoles Catalyst | Rate (mol/kg/h) | Triethylamine Conversion (%) | Triethylammonium Formate Concentration mmol $g^{-1}$ | | Metal Partitioning Ratio |
|---|---|---|---|---|---|---|---|
| | | | | | Aqueous Phase | Organic Phase | Organic:Aqueous |
| 14 | $RhCl_3/PBu_3$ | 1.14 | 5.32 | 68.40 | 3.29 | 0.06 | 432/1 |
| 15 | $RhCl_3/PBu_3$ | | 6.83 | 61.90 | 2.68 | 0.05 | |
| 16 | $RhCl_3/PBu_3$ | | 7.70 | 73.20 | 3.09 | 0.05 | |
| 17 | $RhCl_3/PBu_3$ | | 8.00 | 75.50 | 3.19 | 0.05 | 262/1 |
| 18 | $RuCl_3/PBu_3$ | 1.13 | 3.71 | 53.80 | 2.72 | 0.07 | |
| 19 | $RuCl_3/PBu_3$ | | 2.31 | 60.30 | 2.50 | 0.07 | |
| 20 | $RuCl_3/PBu_3$ | | 0.69 | 64.80 | 2.75 | 0.06 | |

## Claims

1. A process for the production of a formate salt of a nitrogenous base which process comprises reacting hydrogen and carbon dioxide with a nitrogenous base in the presence of a catalyst comprising an

inorganic or organometallic compound of a metal of Group VIII of the Periodic Table and a compound of an element of Group VA of the Periodic Table of the Elements - wherein the process is operated in a two phase system comprising two substantially immiscible solvents, a first solvent which preferentially dissolves the catalyst and a second solvent which preferentially dissolves the formate salt of the nitrogenous base, and separating the first solvent phase containing dissolved catalyst from the second solvent phase containing dissolved formate salt of the nitrogenous base.

**2.** A process according to claim 1 wherein the first solvent is an inert organic solvent, which is either an aliphatic or aromatic hydrocarbon solvent.

**3.** A process according to claim 2 wherein the inert organic solvent is either heptane or toluene.

**4.** A process according to claim 2 wherein the first solvent is a hydrocarbyl phosphine.

**5.** A process according to claim 4 wherein the hydrocarbyl phosphine is of the formula:-
either

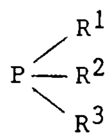

(I)

or

$R^1R^2P(CH_2)_nPR^3R^4$ (II)

or

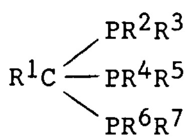

(III)

wherein in the formulae (I) to (III) $R^1$ to $R^7$ are independently either hydrogen or hydrocarbyl groups containing from 1 to 20 carbon atoms and n is an integer from 1 to 10, or any two of $R^1$ to $R^7$ may together form an organic cyclic ring system bound to phosphorus.

**6.** A process according to either claim 4 or claim 5 wherein the first solvent is tri-n-butyl phosphine.

**7.** A process according to any one of claims 1 to 6 wherein the second solvent is either water, glycerol, or water in admixture with a solvent comprising an alcohol, a glycol, a polyol, a sulpholane or a mixture of at least two thereof.

**8.** A process according to any one of claims 1 to 6 wherein the second solvent is water.

**9.** A process according to any one of the preceding claims wherein the nitrogenous base is one containing a tertiary nitrogen atom and is of the formula:
either

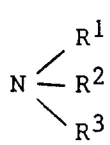

(IV)

or

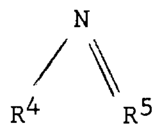

(V)

wherein in the formulae (IV) and (V) $R^1$, $R^2$ and $R^3$, which may be the same or different, are hydrocarbyl groups or substituted hydrocarbyl groups or any two or all of $R^1$, $R^2$ and $R^3$ form part of a ring, $R^4$ is a hydrocarbyl group or substituted hydrocarbyl group and $R^5$ is a divalent organic group or $R^4$ and $R^5$ form part of a ring.

**10.** A process according to claim 9 wherein the nitrogenous base is a $C_1$ to $C_{10}$ trialkylamine.

**11.** A process according to any one of the preceding claims wherein in the catalyst the metal of Group VIII is either rhodium or ruthenium.

**12.** A process according to any one of the preceding claims wherein in the catalyst there is used an organophosphorus compound.

**13.** A process according to claim 12 wherein the organophosphorus compound is an organophosphorus compound used as the solvent.

**14.** A process according to any one of the preceding claims wherein the reaction is effected at a temperature in the range from 15 to 200 °C.

**15.** A process for the production of formic acid which comprises the steps of:-

(1) reacting hydrogen, carbon dioxide and a first nitrogenous base in the presence as catalyst of an inorganic or organometallic compound of a metal of Group VIII of the Periodic Table to produce a formate salt of the first nitrogenous base in a two-phase liquid system comprising two substantially immiscible solvents, the first solvent being one which preferentially dissolves the catalyst and the second solvent being one which preferentially dissolves the formate salt of the first nitrogenous base,

(2) separating the first solvent phase containing dissolved catalyst from the second solvent phase containing dissolved formate salt of the first nitrogenous base,

(3) recycling the first solvent containing dissolved catalyst in whole or in part to step (1),

(4) reacting the formate salt of the first nitrogenous base, either dissolved in the second solvent or separated therefrom, with a second nitrogenous base which is (i) weaker than the first base, and (ii) is less volatile than the first base under conditions to cause the second base to displace the first base and thereby form a formate salt of the second nitrogenous base which is thermally decomposable at a temperature higher than the boiling point of the first base,

(5) separating the formate salt of the second nitrogenous base from the first nitrogenous base, and

(6) thermally decomposing the formate salt of the second nitrogenous base separated in step (5) to yield formic acid and the second nitrogenous base.

**16.** A process according to claim 15 wherein in step (1) the first nitrogenous base is a $C_1$ to $C_{10}$ trialkylamine, the metal of Group VIII is ruthenium, the first solvent is tri-n-butyl phosphine and the second solvent is water.

**17.** A process according to either claim 15 or claim 16 wherein in step (2) the first solvent phase is separated from the second solvent phase by decantation.

**18.** A process according to any one of claims 15 to 17 wherein in step (4) the formate salt of the first nitrogenous base after separation from the second solvent is reacted with the second nitrogenous base which is an imidazole of the general formula:

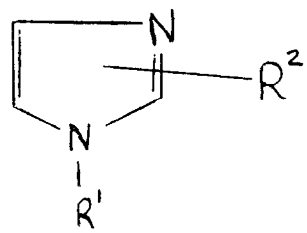

(VI)

wherein in the formula (VI) $R^1$ is a monovalent hydrocarbon group containing 1 to 12 carbon atoms and $R^2$ is a hydrogen atom or an $R^1$ group, the total number of carbon atoms in $R^1$ and $R^2$ being from 4 to 12.

**Patentansprüche**

1. Verfahren zur Herstellung eines Formiatsalzes einer Stickstoffbase durch Umsetzen von Wasserstoff und Kohlendioxid mit einer Stickstoffbase in Gegenwart eines eine anorganische oder Organometallverbindung eines Metalls der Gruppe VIII des Periodensystems und eine Verbindung eines Elements der Gruppe VA des Periodensystems der Elemente umfassenden Katalysators, wobei das Verfahren in einem Zweiphasensystem mit zwei im wesentlichen nicht mischbaren Lösungsmitteln - einem ersten Lösungsmittel, das vorzugsweise den Katalysator löst, und einem zweiten Lösungsmittel, das vorzugsweise das Formiatsalz der Stickstoffbase löst - durchgeführt wird, und Abtrennen der ersten Lösungsmittelphase mit dem darin gelösten Katalysator von der zweiten Lösungsmittelphase mit dem darin gelösten Formiatsalz der Stickstoffbase.

2. Verfahren nach Anspruch 1, wobei das erste Lösungsmittel aus einem inerten organischen Lösungsmittel besteht, bei dem es sich entweder um ein aliphatisches oder aromatisches Kohlenwasserstofflösungsmittel handelt.

3. Verfahren nach Anspruch 2, wobei das inerte organische Lösungsmittel entweder aus Heptan oder Toluol besteht.

4. Verfahren nach Anspruch 2, wobei das erste Lösungsmittel aus einem Kohlenwasserstoffphosphin besteht.

5. Verfahren nach Anspruch 4, wobei das Kohlenwasserstoffphosphin einer der folgenden Formeln entspricht:

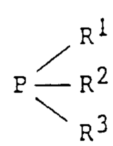

(I)

oder

$R^1R^2P(CH_2)_nPR^3R^4$ (II)

oder

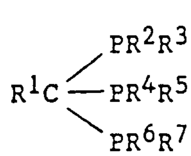

(III)

worin (in den Formeln I bis III) $R^1$ bis $R^7$ unabhängig voneinander entweder Wasserstoff oder

Kohlenwasserstoffgruppen mit 1 bis 20 Kohlenstoffatom(en) bedeuten und n für eine ganze Zahl von 1 bis 10 steht oder irgendwelche zwei Reste $R^1$ bis $R^7$ zusammen ein an Phosphor gebundenes organisches cyclisches Ringsystem bilden können.

**6.** Verfahren nach Anspruch 4 oder Anspruch 5, wobei das erste Lösungsmittel aus Tri-n-butylphosphin besteht.

**7.** Verfahren nach einem der Ansprüche 1 bis 6, wobei das zweite Lösungsmittel entweder aus Wasser, Glyzerin oder Wasser im Gemisch mit einem einen Alkohol, ein Glykol, ein Polyol, ein Sulfolan oder ein Gemisch von mindestens zwei derselben umfassenden Lösungsmittel besteht.

**8.** Verfahren nach einem der Ansprüche 1 bis 6, wobei das zweite Lösungsmittel Wasser ist.

**9.** Verfahren nach einem der vorhergehenden Ansprüche, wobei die Stickstoffbase aus einer mit einem tertiären Stickstoffatom besteht und einer der folgenden Formeln entspricht:

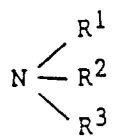

(IV)

oder

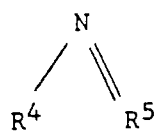

(V)

worin (in den Formeln IV und V) $R^1$, $R^2$ und $R^3$, die gleich oder verschieden sein können, für Kohlenwasserstoffgruppen oder substituierte Kohlenwasserstoffgruppen stehen oder beliebige zwei oder alle Reste $R^1$, $R^2$ und $R^3$ einen Teil eines Rings bilden, $R^4$ eine Kohlenwasserstoffgruppe oder eine substituierte Kohlenwasserstoffgruppe bedeutet und $R^5$ eine zweiwertige organische Gruppe darstellt oder $R^4$ und $R^5$ einen Teil eines Rings bilden.

**10.** Verfahren nach Anspruch 9, wobei die Stickstoffbase aus einem $C_1$-$C_{10}$-Trialkylamin besteht.

**11.** Verfahren nach einem der vorhergehenden Ansprüche, wobei in dem Katalysator das Metall der Gruppe VIII entweder aus Rhodium oder aus Ruthenium besteht.

**12.** Verfahren nach einem der vorhergehenden Ansprüche, wobei in dem Katalysator eine Organophosphorverbindung verwendet wird.

**13.** Verfahren nach Anspruch 12, wobei die Organophosphorverbindung aus einer als das Lösungsmittel verwendeten Organophosphorverbindung besteht.

**14.** Verfahren nach einem der vorhergehenden Ansprüche, wobei die Reaktion bei einer Temperatur im Bereich von 15 bis 200 °C durchgeführt wird.

**15.** Verfahren zur Herstellung von Ameisensäure, umfassend die folgenden Schritte:
(1)Umsetzen von Wasserstoff, Kohlendioxid und einer ersten Stickstoffbase in Gegenwart einer anorganischen oder Organometallverbindung eines Metalls der Gruppe VIII des Periodensystems als Katalysator zur Herstellung eines Formiatsalzes der ersten Stickstoffbase in einem Zweiphasenflüssigkeitssystem mit zwei im wesentlichen nicht mischbaren Lösungsmitteln, wobei es sich bei dem ersten Lösungsmittel um eines handelt, das vorzugsweise den Katalysator löst, und wobei es sich bei dem zweiten Lösungsmittel um eines handelt, das vorzugsweise das Formiatsalz der ersten Stickstoffbase löst,

(2)Abtrennen der ersten Lösungsmittelphase mit dem darin gelösten Katalysator von der zweiten Lösungsmittelphase mit dem darin gelösten Formiatsalz der ersten Stickstoffbase,

(3)vollständiges oder teilweises Rückführen des ersten Lösungsmittels mit dem darin gelösten Katalysator in Stufe (1),

(4)Umsetzen des Formiatsalzes der ersten Stickstoffbase entweder in Lösung in dem zweiten Lösungsmittel oder in abgetrennter Form davon mit einer zweiten Stickstoffbase, die (i) schwächer als die erste Base und (ii) weniger flüchtig als die erste Base ist, unter Bedingungen, um ein Ersetzen der ersten Base durch die zweite Base zu bedingen, um dadurch ein Formiatsalz der zweiten Stickstoffbase, das thermisch bei einer Temperatur über dem Siedepunkt der ersten Base zersetzbar ist, zu bilden,

(5)Abtrennen des Formiatsalzes der zweiten Stickstoffbase von der ersten Stickstoffbase und

(6)thermisches Zersetzen des in Stufe (5) abgetrennten Formiatsalzes der zweiten Stickstoffbase unter Bildung von Ameisensäure und der zweiten Stickstoffbase.

**16.** Verfahren nach Anspruch 15, wobei in Stufe 1 die erste Stickstoffbase aus einem $C_1$-$C_{10}$-Trialkylamin besteht, das Metall der Gruppe VIII Ruthenium ist und es sich bei dem ersten Lösungsmittel um Tri-n-butylphosphin und bei dem zweiten Lösungsmittel um Wasser handelt.

**17.** Verfahren nach Anspruch 15 oder Anspruch 16, wobei in Stufe (2) die erste Lösungsmittelphase von der zweiten Lösungsmittelphase durch Dekantieren abgetrennt wird.

**18.** Verfahren nach einem der Ansprüche 15 bis 17, wobei in Stufe (4) das Formiatsalz der Stickstoffbase nach Abtrennung von dem zweiten Lösungsmittel mit der zweiten Stickstoffbase, bei der es sich um ein Imidazol der folgenden allgemeinen Formel handelt:

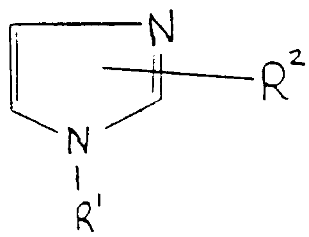

(VI)

worin in Formel (VI) $R^1$ für eine einwertige Kohlenwasserstoffgruppe mit 1 bis 12 Kohlenstoffatom(en) steht und $R^2$ ein Wasserstoffatom oder eine $R^1$-Gruppe bedeutet, wobei die Gesamtzahl der Kohlenstoffatome in $R^1$ und $R^2$ 4 bis 12 beträgt, umgesetzt wird.

## Revendications

**1.** Procédé pour la production d'un formiate d'une base azotée, procédé qui comprend la réaction d'hydrogène et d'anhydride carbonique avec une base azotée en présence d'un catalyseur comprenant un composé inorganique ou organométallique d'un métal du Groupe VIII du Tableau Périodique et un composé d'un élément du Groupe VA du Tableau Périodique des Eléments - ledit procédé étant mis en oeuvre dans un milieu biphasique comprenant deux solvants pratiquement non miscibles, consistant en un premier solvant qui dissout préférentiellement le catalyseur et un second solvant qui dissout préférentiellement le formiate de la base azotée, et la séparation de la première phase de solvant contenant le catalyseur dissous de la seconde phase de solvant contenant le formiate dissous de la base azotée.

**2.** Procédé suivant la revendication 1, dans lequel le premier solvant est un solvant organique inerte, qui est un solvant hydrocarboné aliphatique ou aromatique.

**3.** Procédé suivant la revendication 2, dans lequel le solvant organique inerte est l'heptane ou le toluène.

**4.** Procédé suivant la revendication 2, dans lequel le premier solvant est une hydrocarbylphosphine.

**5.** Procédé suivant la revendication 4, dans lequel l'hydrocarbylphosphine répond à la formule :

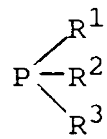

(I)

ou

$R^1R^2P(CH_2)_nPR^3R^4$ (II)

ou bien

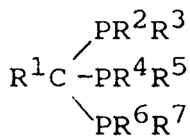

(III)

dans laquelle $R^1$ à $R^7$ représentent indépendamment l'hydrogène ou des groupes hydrocarbyle contenant 1 à 20 atomes de carbone et n est un nombre entier de 1 à 10, ou bien deux quelconques de $R^1$ à $R^7$ peuvent former conjointement un système cyclique organique lié à l'atome de phosphore.

**6.** Procédé suivant la revendication 4 ou la revendication 5, dans lequel premier solvant est la tri-n-butylphosphine.

**7.** Procédé suivant l'une quelconque des revendications 1 à 6, dans lequel le second solvant est l'eau, le glycérol ou de l'eau en mélange avec un solvant comprenant un alcool, un glycol, un polyol, un sulfolane ou un mélange d'au moins deux de ces composés.

**8.** Procédé suivant l'une quelconque des revendications 1 à 6, dans lequel le second solvant est l'eau.

**9.** Procédé suivant l'une quelconque des revendications précédentes, dans lequel la base azotée est une base azotée contenant un atome d'azote tertiaire et répond à la formule :

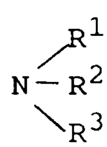

(IV)

ou

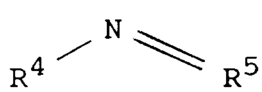

(V)

dans laquelle $R^1$, $R^2$ et $R^3$, qui peuvent être identiques ou différents, représentent des groupes hydrocarbyle ou des groupes hydrocarbyle substitués ou bien deux quelconques ou la totalité de $R^1$, $R^2$ et $R^3$ font partie d'un noyau, $R^4$ représente un groupe hydrocarbyle ou un groupe hydrocarbyle substitué et $R^5$ représente un groupe organique divalent ou bien $R^4$ et $R^5$ font partie d'un noyau.

**10.** Procédé suivant la revendication 9, dans lequel la base azotée est une tri(alkyle en $C_1$ à $C_{10}$)-amine.

**11.** Procédé suivant l'une quelconque des revendications précédentes, dans lequel, dans le catalyseur, le métal du Groupe VIII est le rhodium ou le ruthénium.

**12.** Procédé suivant l'une quelconque des revendications précédentes, dans lequel, dans le catalyseur, un composé organique de phosphore est utilisé.

**13.** Procédé suivant la revendication 12, dans lequel le composé organique de phosphore est un composé organique de phosphore utilisé comme solvant.

**14.** Procédé suivant l'une quelconque des revendications précédentes, dans lequel la réaction est conduite à une température comprise dans l'intervalle de 15 à 200 °C.

**15.** Procédé de production d'acide formique, qui comprend les étapes :

(1) de réaction d'hydrogène, d'anhydride carbonique et d'une première base azotée en présence, comme catalyseur, d'un composé inorganique ou organométallique d'un métal du Groupe VIII du Tableau Périodique pour produire un formiate de la première base azotée dans un milieu liquide biphasique comprenant deux solvants pratiquement non miscibles, le premier solvant étant un solvant qui dissout préférentiellement le catalyseur et le second solvant étant un solvant qui dissout préférentiellement le formiate de la première base azotée,

(2) de séparation de la première phase de solvant contenant le catalyseur dissous de la seconde phase de solvant contenant le formiate dissous de la première base azotée,

(3) de recyclage du premier solvant contenant le catalyseur dissous en totalité ou en partie à l'étape (1),

(4) de réaction du formiate de la première base azotée, à l'état dissous dans le second solvant ou bien séparé de ce solvant, avec une seconde base azotée qui est (i) plus faible que la première base et (ii) moins volatile que la première base dans des conditions provoquant le déplacement de la première base par la seconde base et ainsi la formation d'un formiate de la seconde base azotée qui peut être décomposé par la chaleur à une température supérieure au point d'ébullition de la première base,

(5) de séparation du formiate de la seconde base azotée de la première base azotée, et

(6) de décomposition thermique du formiate de la seconde base azotée séparé dans l'étape (5), ce qui donne de l'acide formique et la seconde base azotée.

**16.** Procédé suivant la revendication 15, dans lequel, dans l'étape (1), la première base azotée est une tri-(alkyle en $C_1$ à $C_{10}$)-amine, le métal du Groupe VIII est le ruthénium, le premier solvant est la tri-n-butylphosphine et le second solvant est l'eau.

**17.** Procédé suivant la revendication 15 ou la revendication 16, dans lequel, dans l'étape (2), la première phase de solvant est séparée de la seconde phase de solvant par décantation.

**18.** Procédé suivant l'une quelconque des revendications 15 à 17, dans lequel, dans l'étape (4), le formiate de la première base azotée, après séparation du second solvant, est amené à réagir avec la seconde base azotée qui est un imidazole de formule générale :

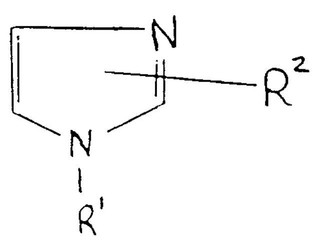

(VI)

dans laquelle $R^1$ représente un groupe hydrocarboné monovalent contenant 1 à 12 atomes de carbone et $R^2$ représente un atome d'hydrogène ou un groupe $R^1$, le nombre total d'atomes de carbone dans $R^1$ et $R^2$ ayant une valeur de 4 à 12.